# EUROPÄISCHE PATENTSCHRIFT

(11) **EP 2 084 543 B1**
(45) Veröffentlichungstag und Bekanntmachung des Hinweises auf die Patenterteilung: **18.10.2017**
(21) Anmeldenummer: 07846265.2
(22) Anmeldetag: 26.10.2007
(51) Int. Cl.: G01N 33/68, G01N 33/74

(54) **RISIKOSTRATIFIZIERUNG DES AKUTEN KORONARSYNDROMS MITTELS FRAGMENTEN / TEILPEPTIDEN DES PROVASOPRESSINS, INSBESONDERE COPEPTIN ODER NEUROPHYSIN II**
RISK STRATIFICATION FOR ACUTE CORONARY SYNDROME BY MEANS OF FRAGMENTS/PARTIAL PEPTIDES OF PROVASOPRESSIN, ESPECIALLY COPEPTIN OR NEUROPHYSIN II
STRATIFICATION DES RISQUES DU SYNDROME CORONARIEN AIGU AU MOYEN DE FRAGMENTS ET DE PEPTIDES PARTIELS DE LA PROVASOPRESSINE, EN PARTICULIER DE LA COPEPTINE OU DE LA NEUROPHYSINE II

(30) Priorität: 26.10.2006 DE 102006050497; 04.12.2006 DE 102006057409
(43) Veröffentlichungstag der Anmeldung: 05.08.2009
(73) Patentinhaber: B.R.A.H.M.S GmbH, 16761 Hennigsdorf (DE)
(72) Erfinder: BERGMANN, Andreas, 12351 Berlin (DE); MORGENTHALER, Nils, 13503 Berlin (DE); PAPASSOTIRIOU, Jana, 14163 Berlin (DE); STRUCK, Joachim, 13465 Berlin (DE); LEONG, L., NG, Leicester LE2 3NB (GB)
(74) Vertreter: Simandi, Claus
(86) Internationale Anmeldenummer: PCT/DE2007/001928
(87) Internationale Veröffentlichungsnummer: WO 2008/049422

(56) Entgegenhaltungen:
- EP-A- 1 363 128
- EP-A- 1 628 136
- WO-A-2004/006860
- KHAN SQ ET AL: "C-terminal proVasopressin (copeptin) as a novel and prognostic marker in acute myocardial indarction - The Leicester Acute Myocardial Infarction Peptide (LAMP) Study" CIRCULATION, AMERICAN HEART ASSOCIATION, DALLAS, TX, PAGE(S) 721, 1. Oktober 2006 (2006-10-01), XP002491231
- KHAN SOHAIL Q ET AL: "C-terminal provasopressin (copeptin) as a novel and prognostic marker in acute myocardial infarction Leicester acute myocardial infarction peptide (LAMP) study" CIRCULATION, Bd. 115, Nr. 16, April 2007 (2007-04), Seiten 2103-2110, XP002491227 ISSN: 0009-7322

## Beschreibung

Die Erfindung betrifft ein Verfahren zur Risikostratifizierung des akuten Koronarsyndroms (ACS), insbesondere des akuten Myokardinfarkts (AMI), wobei eine Bestimmung von Copeptin oder Neurophysin II mittels einer in-vitro Diagnose erfolgt. Ferner betrifft die Erfindung hierzu geeignete Kombinationen von Biomarkern zur in-vitro Diagnostik.

Die Risikostratifizierung hat eine zunehmende Bedeutung im Bereich der Herzerkrankungen, sei es bei symptomatischen oder asymptomatischen Patienten. Insbesondere auf dem Gebiet des akuten Koronarsyndroms besteht ein hohes Bedürfnis an einer geeigneten Risikostratifizierung.

Die Risikostratifizierung dient zum Auffinden von Patienten mit der schlechteren Prognose, zwecks intensiverer Diagnostik und Therapie/Behandlung mit dem Ziel eines möglichst günstigen Verlaufs zu ermöglichen. Eine geeignete Risikostratifizierung sollte demnach effektive Behandlungsverfahren nach sich ziehen, die beim akuten Koronarsyndrom mit den perkutanen Koronarinterventionen und den neueren Arzneimitteln gegeben sind.

Zwecks einer geeigneten Therapie, bedarf es einer frühen Diagnose und Differenzierung des akuten Koronarsyndroms bereits in der Notaufnahme in Verbindung mit der Notwendigkeit klinische Entscheidungen zu treffen. Aufgrund unspezifischer Symptome (Brustschmerzen) bei akutem Koronarsyndrom ist sowohl die Differenzierung und Abgrenzung von anderen Erkrankungen als auch die Erkennung des akuten Koronarsyndroms wesentlich.

Im Stand der Technik sind biochemische Marker - insbesondere die Klassiker wie kardiale Troponine, Myoglobin und CK-MB-Masse - zur Prognose eines Myokardinfarkts angestrengt worden (Katus, H. A.; Remppis, A.; Scheffold, T.; Diederich, K. W. und Kuebler, W. (1991): Intracellular compartmentation of cardiac troponin T and its release kinetics in patients with reperfused and nonreperfused myocardial infarction, Am J Cardiol 67 (16): 1360-1367). Als ein weiterer effektiver biochemischer Marker hat sich in der Myokarddiagnostik das B-Typ natriuretischem Peptid (BNP) samt Pro-BNP (NT-ProBNP) (EP1363128B1, EP1666881A2) erwiesen.

Copeptin (auch: C-terminales proAVP) ist in WO 2006/018315 (BRAHMS AG) als Biomarker zur in-vitro Diagnose von AMI beschrieben. Ein entsprechender Copeptin-Assay wird in Morgenthaler et al. (Nils G. Morgenthaler, Joachim Struck, Christine Alonso and Andreas Bergmann, Assay for the Measurement of Copeptin, a Stable Peptide Derived from the Precursor of Vasopressin Clinical Chemistry 52: 112-119, 2006) offenbart.

EP 1 628 136 A1 beschreibt die potenzielle Eignung und Verwendung von Copeptin zur in-vitro-Diagnose von kardiovaskulären Erkrankungen und Herzerkrankungen.

EP 1 363 128 A1 offenbart eine Markerkombination zur Diagnose des akuten Koronarsyndroms, welche jedoch Copeptin nicht enthält.

Morgenthaler N. G. et al. ,,Assay for the measurement of copeptin, a stable peptide derived from the precursor of vasopressin", CLINICAL CHEMISTRY, AMERICAN ASSOCIATION FOR CLINICAL CHEMISTRY, WASHINGTON, DC, Bd. 52, Nr. 1, 1. Januar 2006, Seiten 112-119, beschreibt die Bestimmung von Copeptin als "surrogate marker" für Arginin-Vasopressin.

Stoiser B. et al "Copeptin, a fragment of the vasopressin precursor, as a novel predictor of outcome in heart failure", EUROPEAN JOURNAL OF CLINICAL INVESTIGATION, WILEYBLACKWELL PUBLlSHING LTD, GB, Bd. 36, Nr. 11, 1. November 2006, Seiten 771-778, beschreibt die potenzielle Eignung und Verwendung von Copeptin zur in-vitro-Diagnose von Herzinsuffizienz, jedoch mit Veröffentlichung nach dem Prioritätsdatum.

Neurophysin ist als Marker bisher beschrieben für die Nikotinaufnahme (Robinson AG. Isolation, assay, and secretion of individual human neurophysins. J Clin Invest 1975;55:360-7), Krebs- und nicht-Krebs-assoziierter SIADH (Syndrome of inappropriate ADH secretion) und nephrogenem Diabetes insipidus (Pullan PT, Clappison BH, Johnston CI. Plasma vasopressin and human neurophysins in physiological and pathological states associated with changes in vasopressin secretion. J Clin Endocrinol Metab 1979;49:580-7; North WG, LaRochelle FT, Jr., Melton J, Mills RC. Isolation and partial characterization of two human neurophysins: their use in the development of specific radioimmunoassays. J Clin Endocrinol Metab 1980; 51:884-91).

Nachteilig an den bekannten Diagnoseverfahren unter Verwendung der bisher bekannten Markern ist jedoch, dass eine frühzeitige und vollständige Erfassung von Risikopatienten nicht gelingt und daher eine Risikostratifizierung nur ungenügend erfolgt. Eine der Erfindung zugrunde liegende Aufgabe besteht daher darin, ein Verfahren zur Risikostratifizierung des akuten Koronarsyndroms zu entwickeln, das eine verbesserte Erfassung von Risikopatienten ermöglicht.
Ferner ist nachteilig, dass im Stand der Technik zumeist keine hinreichende Sensitivität und/oder Spezifität der Marker erreicht wird.
Eine weitere Aufgabe besteht daher darin, ein Verfahren zur Risikostratifizierung des akuten Koronarsyndroms bereitzustellen, wobei mindestens ein Marker oder eine Kombination von Marker eine hinreichende Sensitivität und Spezifität in einer in-vitro Diagnose aufweist.

Daher ist es Aufgabe der vorliegenden Erfindung ein Verfahren zur Risikostratifizierung des akuten Koronarsyndroms bereitzustellen. Die Aufgabe wird durch ein Verfahren zur Risikostratifizierung des akuten Koronarsyndroms gelöst, wobei eine Bestimmung Copeptin oder Neurophysin II mittels einer in-vitro Diagnose erfolgt (nachstehend erfindungsgemäßes Verfahren).

Überraschender Weise weisen Copeptin oder Neurophysin II eine hohe Sensitivität und Spezifität für die Diagnose des akuten Koronarsyndroms auf (Siehe Beispiele und Figuren).

Der Begriff "akutes Koronarsyndrom" umfasst verschiedene Phasen der koronaren Herzerkrankung, die unmittelbar lebensbedrohlich sind. Dies betrifft insbesondere die Notfallmedizin, und zwar einen akuten Myokardinfarkt und / oder Angina pectoris sowie einen plötzlichen Herztod. Neben dem akuten Myokardinfarkt, der nach WHO-Kriterien (WHO (1979): Nomenclature and criteria for diagnosis of ischemic heart disease. Report of the Joint International Society and Federation of Cardiology/World Health Organization task force on standardization of clinical nomenclature, Circulation 59 (3): 607-609) definiert ist als akutes, länger als 20 Minuten andauerndes Brustschmerzereignis, verbunden mit ST-Streckenhebungen und/oder einer Erhöhung myokardialer Enzyme, wurde der Begriff der instabilen Angina pectoris (AP) geprägt, die erfindungsgemäß unter "akutes Koronarsyndrom" zu lesen sind (Hamm CW: Leitlinien: Akutes Koronarsyndrom (ACS) - Teil 1: ACS ohne persistierende ST-Hebung. Z Kardiol (2004) 93:72-90; siehe auch: Pschyrembel, De Gruyter, Berlin 2004).

Der Begriff "Risikostratifizierung" umfasst erfindungsgemäß das Auffinden von Patienten, insbesondere Notfallpatienten und Risikopatienten, mit der schlechteren Prognose, zwecks intensiverer Diagnostik und Therapie/Behandlung des akuten Koronarsyndroms, insbesondere Myokardinfekt, mit dem Ziel einen möglichst günstigen Verlauf zu ermöglichen. Eine erfindungsgemäße Risikostratifizierung erlaubt in Folge ein effektives Behandlungsverfahren, die beim akuten Koronarsyndrom mit den perkutanen Koronarinterventionen und den neueren Arzneimitteln gegeben sind.

Daher betrifft die Erfindung ebenfalls die Identifizierung von Patienten mit erhöhtem Risiko oder/und einer ungünstigen Prognose eines akuten Koronarsyndroms, insbesondere Myokardinfekts, und zwar bei symptomatischen und / oder asymptomatischen Patienten, insbesondere Notfallpatienten.

Besonders vorteilhaft kann insbesondere in Fällen der Notfall- und /oder Intensivmedizin mittels des erfindungsgemäßen Verfahren eine sichere Stratifizierung erfolgen. Das erfindungsgemäße Verfahren ermöglicht daher klinische Entscheidungen, die zu einem schnellen Therapieerfolg und zur Vermeidung von Todesfällen führen. Solche klinische Entscheidungen umfassen ebenfalls weiterführende Behandlung mittels Arzneimitteln zur Behandlung oder Therapie des akuten Koronarsyndroms, insbesondere Myokardinfarkt (AMI) und der Angina pectoris (AP).

Daher betrifft die Erfindung ebenfalls ein Verfahren zur Risikostratifizierung von Patienten mit einem akuten Koronarsyndrom zur Durchführung von klinischen Entscheidungen, wie weiterführende Behandlung und Therapie mittels Arzneimitteln, vorzugsweise in der zeitlich kritischen Intensivmedizin oder Notfallmedizin.

In einer weiteren bevorzugten Ausführungsform betrifft das erfindungsgemäße Verfahren daher die Therapiesteuerung des akuten Koronarsyndroms, insbesondere Myokardinfarkt (AMI).

In einer weiteren bevorzugten Ausführungsform des erfindungsgemäßen Verfahrens erfolgt die Risikostratifizierung zur Prognose, zur differentialdiagnostischen Früherkennung und Erkennung, zur Beurteilung des Schweregrades und zur therapiebegleitenden Verlaufsbeurteilung.

In einer weiteren bevorzugten Ausführungsform betrifft die Erfindung ein Verfahren zur in-vitro Diagnostik zur Früh- oder Differentialdiagnose oder Prognose von Myokardinfarkt oder Angina Pectoris, wobei eine Bestimmung von Copeptin oder Neurophysin II an einem zu untersuchenden Patienten durchgeführt wird.

Ferner betrifft die Erfindung ein Verfahren zur Risikostratifizierung des akuten Koronarsyndroms oder ein Verfahren zur in-vitro Diagnostik zur Früh- oder Differentialdiagnose oder Prognose von Myokardinfarkt nach einem der obigen Ausführungsformen, wobei nach Eintreten der Symptome ein cut-off (Schwellenwert) Bereich von 6-20 pmol/L von Copeptin oder Neurophysin II signifikant (spezifisch) für die Diagnose und / oder Risikostratifizierung ist. Weiterhin bevorzugt ist ein cut-off (Schwellenwert) von 6-10 pmol/L, insbesondere 7,5 pmol/L, vorzugsweise bis 2 Stunden nach Eintreten der Symptome.

Ferner betrifft die Erfindung ein Verfahren zur Risikostratifizierung des akuten Koronarsyndroms oder ein Verfahren zur in-vitro Diagnostik zur Früh- oder Differentialdiagnose oder Prognose von Myokardinfarkt nach einem der obigen Ausführungsformen, wobei nach Eintreten der Symptome ein cut-off (Schwellenwert) Bereich von 10-30 pmol/L der Marker Copeptin oder Neurophysin II signifikant (spezifisch) für die Prognose und / oder Risikostratifizierung ist. Weiterhin bevorzugt ist ein cut-off (Schwellenwert) von 10-20 pmol/L. Aus dieser Basis sind diese erfindungsgemäßen Verfahren vorteilhaft sensitiv.

In einer Ausführungsform des erfindungsgemäßen Verfahrens wird dem zu untersuchenden Patienten Körperflüssigkeit, insbesondere Blut entnommen, wahlweise Vollblut oder Serum oder erhältliches Plasma und die Diagnose erfolgt *in vitro*/*ex vivo,* d.h. außerhalb des menschlichen oder tierischen Körpers. Aufgrund der Bestimmung des Markers Provasopressins (proAVP) oder Fragmenten und Teilpeptiden davon, insbesondere Copeptin oder Neurophysin II wird eine hohe Sensitivität und Spezifität für das akute Koronarsyndrom, Myokardinfarkt und Angina Pectoris erzielt und anhand der vorhandenen Menge in mindestens einer Patientenprobe kann die Diagnose oder Risikostratifizierung erfolgen. Ganz besonders bevorzugt ist jedoch der Marker Copeptin (stabiles Fragment aus proAVP bzw. Preprovasopressin). Ebenfalls besonders bevorzugt ist der Marker Neurophysin (stabiles Fragment aus proAVP bzw. Preprovasopressin)

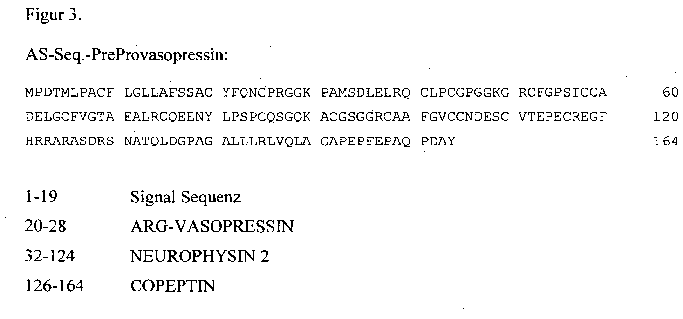

Im Rahmen dieser Erfindung wird unter "Provasopressin" ein humanes Protein oder Polypeptid verstanden, dass aus dem Preprovasopressin erhalten werden kann und im Rahmen des Preprovasopressins die Aminosäuren 29 - 164 umfasst (Siehe ebenfalls WO2006/018315 und Figur 3) und daraus erhältliche Fragmente, nämlich Copeptin (Fragment: AS 126-164 (39AS: SEQ: ASDRSNATQL DGPAGALLLR LVQLAGAPEP FEPAQPDAY) oder Neurophysin II (Fragment: AS 32-124 des Preprovasopressins (93AS: SEQ: AMSDLELRQC LPCGPGGKGR CFGPSICCAD ELGCFVGTAE ALRCQEENYL PSPCQSGQKA CGSGGRCAAF GVCCNDESCV TEPECREGFH RRA). Ferner können diese erfindungsgemäße Polypeptide posttranslationale Modifikationen aufweisen, wie Glykolisierung, Lip(o)idisierung oder Derivatisierungen.

In einer weiteren bevorzugten Ausführungsform betrifft die Erfindung die Diagnose und / oder Risikostratifizierung und /oder Früh- oder Differentialdiagnose und / oder Prognose des akuten Koronarsyndroms, insbesondere Myokardinfarkt (AMI), wobei eine Bestimmung von Neurophysin an einem zu untersuchenden Patienten durchgeführt wird.

In einer weiteren Ausführungsform kann die Bestimmung von Copeptin oder Neurophysin II zusätzlich mit weiteren Markern erfolgen und zwar vorzugsweise solche, die bereits auf ein akutes Koronarsyndrom, insbesondere Myokardinfarkt hinweisen.

Daher betrifft die Erfindung eine solche Ausführungsform des erfindungsgemäßen Verfahrens, wobei die Bestimmung zusätzlich mit mindestens einem weiteren Marker ausgewählt aus der Gruppe inflammatorischer Marker, cardiovaskulärer Marker, neurohormonaler Marker oder ischämischer Marker an einem zu untersuchenden Patienten durchgeführt wird.

Erfindungsgemäß kann der inflammatorische Marker aus mindestens einem Marker aus der Gruppe C-reaktivem Protein (CRP), Cytokinen, wie etwa TNF-alpha, Interleukinen, wie etwa IL-6, Procalcitonin (1-116, 3-116) und Adhäsionsmolekülen, wie VCAM oder ICAM ausgewählt sein sowie der cardiovaskuläre Marker, insbesondere ein Nekrose von Herzmuskelgewebe anzeigenden Marker und den Blutdruck beeinflussenden Marker aus mindestens einem Marker aus der Gruppe Kreatinkinase, Myoglobin, Myeloperoxidase, natriuretisches Protein, insbesondere ANP (bzw. ANF), proANP, NT-proANP, BNP, proBNP, NT-proBNP oder jeweils eine Teilsequenz davon, kardiale Troponin, CRP ausgewählt sein. Ferner werden hierunter ebenfalls Kreislaufregulierende (Pro)Hormone verstanden, insbesondere wie pro-Gastrin-Releasing Peptid (proGRP), pro-Endothelin-1, pro-Leptin, pro-Neuropeptid-Y, pro-Somatostatin, pro- Neuropeptid-YY, pro-Opiomelanocortin oder pro-Adrenomedullin (proADM) oder jeweils eine Teilsequenz davon. Der ischämische Marker kann aus mindestens einem Marker aus der Gruppe Troponin I und T, CK-MB ausgewählt sein. Darüber hinaus kann der neurohormonaler Marker mindestens ein natriuretisches Protein sein, insbesondere ANP (bzw. ANF), proANP, NT-proANP, BNP, proBNP, NT-proBNP oder jeweils eine Teilsequenz davon.

In einer besonders bevorzugten Ausführungsform betrifft die Erfindung eine besonders vorteilhafte Kombination von Biomarkern Copeptin oder Neurophysin II mit natriuretischen Proteinen, insbesondere ANP (bzw. ANF), proANP, NT-proANP, BNP, proBNP, NT-proBNP.

Daher betrifft die Erfindung ein Verfahren zur in-vitro Diagnose des akuten Koronarsyndroms oder Myokardinfarkt, wobei eine Bestimmung von Copeptin oder Neurophysin II in Kombination mit natriuretischen Proteinen, insbesondere ANP (bzw. ANF), proANP, NT-proANP, BNP, proBNP, NT-proBNP oder jeweils eine Teilsequenz davon, an einem zu untersuchenden Patienten durchgeführt wird. Besonders bevorzugt ist wiederum eine Kombination aus Neurophysin II, Copeptin und BNP, proBNP, NT-proBNP, insbesondere Copeptin und proBNP.
Besonders vorteilhaft weisen diese genannten Biomarkerkombinationen Synergien auf, die zu einer verbesserten Spezifität und Sensitivität zur Diagnose des führen (siehe Beispiele).

In einer weiteren Ausführungsform der Erfindung kann das erfindungsgemäße Verfahren im Rahmen einer in-vitro Diagnose mittels parallelen oder simultanen Bestimmungen der Marker durchgeführt werden (z.B. Multititerplatten mit 96 und mehr Kavitäten), wobei die Bestimmungen an mindestens einer Patientenprobe durchgeführt werden.

Ferner kann das erfindungsgemäße Verfahren und dessen Bestimmungen in einer diagnostischen Vorrichtung anhand eines Analyseautomaten, insbesondere mittels einem Kryptor (http://www.kryptor.net/) durchgeführt werden.

In einer weiteren Ausführungsform kann das erfindungsgemäße Verfahren und dessen Bestimmungen mittels einem Schnelltest durchgeführt werden (z.B. lateral-flow Test), sei es in Einzel- oder Multiparameterbestimmung. In einer besonders bevorzugten Ausführungsform handelt es sich um einen Selbsttest oder um eine Vorrichtung, die in der Notfalldiagnostik geeignet ist.

Ferner betrifft die Erfindung die Verwendung von Copeptin oder Neurophysin II zur Risikostratifizierung des akuten Koronarsyndroms oder Myokardinfarkt und/oder zur in-vitro Diagnostik zur Früh- oder Differentialdiagnose oder Prognose von Myokardinfarkt.

In einer besonderen Ausführungsform betrifft die Erfindung die Verwendung Copeptin oder Neurophysin II in Kombination mit natriuretischen Proteinen, insbesondere ANP (bzw. ANF), proANP, NT-proANP, BNP, proBNP, NT-proBNP oder jeweils eine Teilsequenz davon, zur Diagnose und / oder Risikostratifizierung des akuten Koronarsyndroms, Myokardinfarkt oder Angina Pectoris.

Eine weitere Aufgabe ist die Bereitstellung einer entsprechenden diagnostischen Vorrichtung zur Durchführung der erfindungsgemäßen Verfahren.

Im Rahmen dieser Erfindung wird unter einer solchen diagnostischen Vorrichtung, insbesondere ein Array oder Assay verstanden (z.B. Immunoassay, ELISA etc.), im weitesten Sinne eine Vorrichtung zur Durchführung der erfindungsgemäßen Verfahren.

Die Erfindung betrifft zudem ein Kit zur Risikostratifizierung des akuten Koronarsyndroms, und/oder Myokardinfarkt, enthaltend Nachweisreagenzien zur Bestimmung von Copeptin oder Neurophysin II ggfs. oben genannten weiteren Marker. Solche Nachweisreagenzien umfassen z.B. Antikörper etc.

In einer besonderen Ausführungsform betrifft die Erfindung ein Kit zur Diagnose und / oder Risikostratifizierung des akuten Koronarsyndroms und/oder Myokardinfarkt, enthaltend Nachweisreagenzien zur Bestimmung von Copeptin oder Neurophysin II in Kombination mit natriuretischen Proteinen, insbesondere ANP (bzw. ANF), proANP, NT-proANP, BNP, proBNP, NT-proBNP oder jeweils eine Teilsequenz davon, ggfs. oben genannten weiteren Marker. Solche Nachweisreagenzien umfassen z.B. Antikörper, etc.

Nachfolgende Beispiele und Figuren dienen zur näheren Erläuterung der Erfindung, jedoch ohne die Erfindung auf diese Beispiele und Figuren zu beschränken. Die Erfindung ist durch die Ansprüche definiert.

### Beispiele und Figuren:

Patienten, die sich mit dem Leitsymptom des Brustschmerzes in der Notaufnahme eines Krankenhauses vorgestellt haben, wurde während der Eingangsuntersuchung eine Blutprobe entnommen.

131 Patienten mit Myokardinfarkt (MI)(Altersmedian: 64,0 Jahre) wurden in einem Zeitraum von insgesamt 180 Tagen nach Eintritt der akuten MI-Symptome beobachtet:

Innerhalb der ersten 6 Stunden nach Symptomauftritt (Brustschmerz) und Krankenhaus-Einlieferung erfolgten 3 Blutabnahmen jeweils innerhalb der Zeiträume 0-2 Stunden-, 2-4 Stunden-, und 4-6 Stunden nach Eintritt der MI-Symptomatik.
Es erfolgte jeweils die Bestimmung von Copeptin und NT-ProBNP.

Nach Hospitalisierung erfolgte innerhalb der ersten 4 Tage nach therapeutischer Interventionen (Standard, s. Braunwald et al, 2002) (Tag 2-5) je 1 Blutabnahme mit nachlaufender Copeptin-Bestimmung).

Nach Entlassung (Median des Krankenausaufenthaltes: 7,2 Tage) erfolgte die Beobachtung der Patienten in einem Zeitraum von 180 Tagen. Als Ereignisse (events) wurden die Entwicklung einer schweren Herzinsuffizienz und/ oder Tod des Patienten erfasst. Von den beobachteten Patienten waren 115 ohne Ereignisse (o.E.) und 16 mit Ereignissen (+E.). Die Altersmediane der Gruppen betrugen 63,8 und 64,5 Jahre.

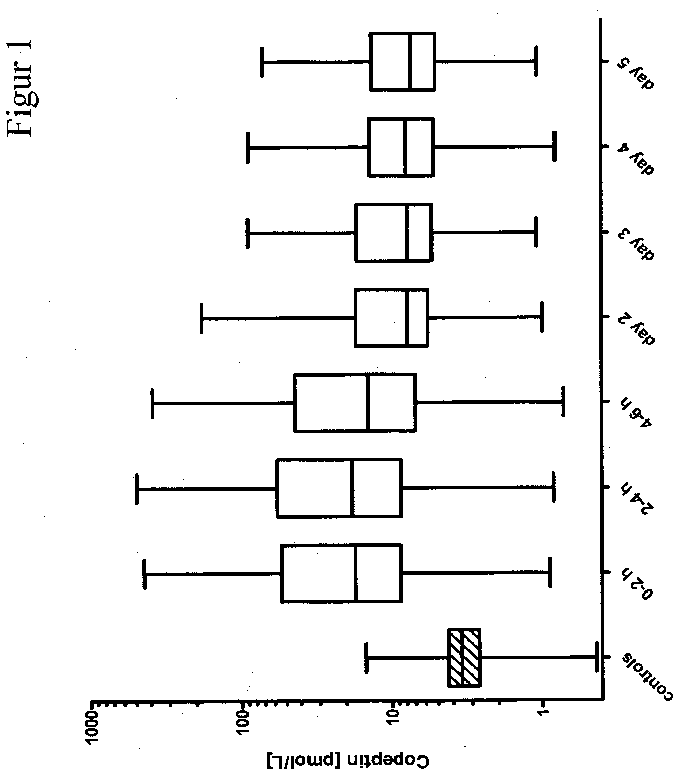

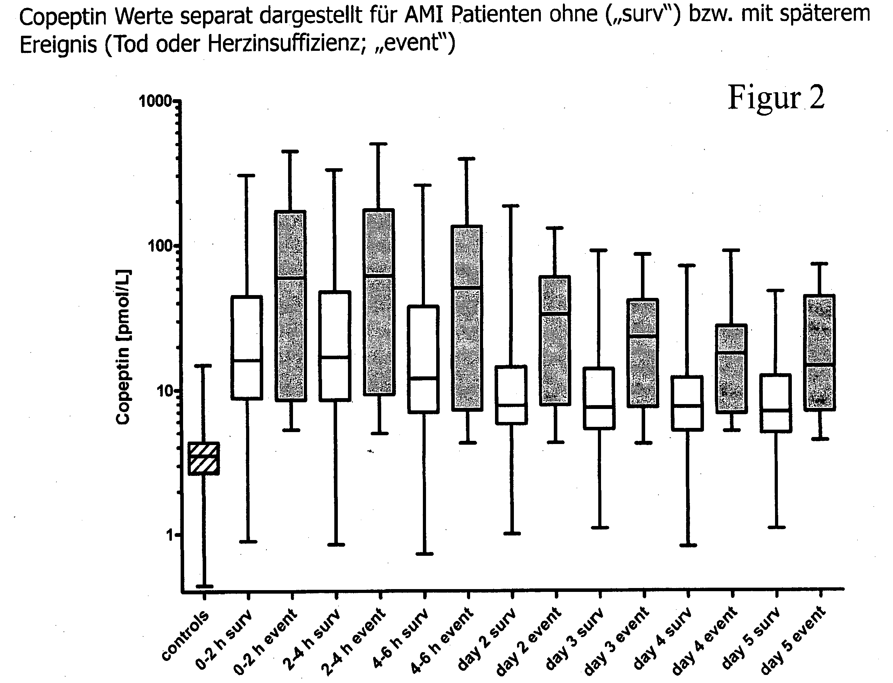

Zur Bewertung der diagnostischen Leistungsfähigkeit der Biomarker für die Frühdiagnose des MI sowie zur Risikostratifizierung erfolgte der Vergleich der Ergebnisse der einzelnen Blutabnahmen bei Patienten mit denen von 200 Menschen (Altermedian: 65,2 Jahre), die keine Anzeichen für einen MI aufzeigten (Kontrollen). Ergebnisse sind in der Tabelle 1 und in den Figuren 1 und 2 dargestellt.

In der Tabelle 1 sind die Sensitivitäten (Werte > Cut Off (Schwellenwert)) bei MI-Patienten gegen die entsprechenden Spezifitäten (Werte < Cut Off (Schwellenswert)) bei den Kontrollen zu den angegebenen Zeiten nach Eintritt der Symptome angegeben.

Schon innerhalb des frühesten Beobachtungszeitraums (0-2 Stunden) im Vergleich zu Kontrollen das Copeptin stark erhöht. Z.B. zeigten nur 4,5% aller Kontrollen einen Copeptin-Wert, der höher als 7,5 pmol/l (exemplarischer Cut Off (Schwellenwert)) liegt (Spezifität= 95,5%), wohingegen 78,3 % der Patienten ohne spätere Ereignisse und sogar 87,5% der Patienten mit Ereignissen Copeptin-Konzentrationen über 7,5 pmol/l aufwiesen (Sensitivität). Im Vergleich zu bislang in der Routine-Diagnostik verwendeten Markern (Troponine, Myoglobin, CK-MB) ergeben sich überraschende Steigerungen der Sensitivitäten zu Zeitpunkten, die nunmehr die Verwendung dieser Biomarker zur Früh- bzw Differentialdiagnose des MI sowie zur Risikostratifizierung des akuten Koronarsyndroms erlaubt. (Troponine; CK-MB erscheinen in der Regel erst nach 6 Stunden post-Ereignis, Myoglobin erscheint mit geringer Sensitivität zwar früh, fällt aber nach ca. 2 Stunden wieder ab, d.h. es ergibt sich ein unsicheres diagnostisches Fenster.) Vorteilhafterweise ist die Copeptin-Erhöhung, d.h. der Zeitpunkt der Blutentnahme zur Frühdiagnostik von untergeordneter Bedeutung (am Cut Off 7,5 pmol/l z.B. 78,3/ 80,0/ 73% Sensitivität bei 0-2/2-4/4-6 Stunden, respektive.

Kombination mit proBNP oder NT-ProBNP Überraschend zeigte sich, dass die Kombination des Copeptin mit proBNP (NT-ProBNP) eine deutliche Steigerung der Sensitivität der Frühdiagnose des MI erlaubt (s. Tabelle 1). Das proBNP (NT-ProBNP) ist der diagnostischen Leistungsfähigkeit des Copeptin zwar unterlegen, erlaubt aber z.B. bei der Bewertung Copeptin > 7,5 pmol/l und/oder proBNP (NT-ProBNP) > 200 pmol/ml bei einer den entsprechenden Einzelmarkern vergleichbaren Spezifität von ca. 95% eine Erhöhung der Sensitivitäten zu allen Zeitpunkten in der Nicht-Ereignisgruppe um 3-6% und in der Ereignisgruppe um 6-9,5% im Vergleich zu den Sensitivitäten des Copeptin als Einzelmarker.

**Tabelle 1:**

| | 0-2 h o.E. | 0-2 h +E. | 2-4 h o.E. | 2-4 h +E. | 4-6 h o.E. | 4-6 h +E | Kontroll en (Spezifi tät) |
|---|---|---|---|---|---|---|---|
| Copeptin (pmol/l) | | | | | | | |
| Sens/Spef für cut-off 6 | 88,7% | 93,8% | 89, 6% | 93,8% | 80,0% | 87,5% | 93,0% |
| Sens/Spef für cut-off 7,5 | 78,3% | 87,5% | 80,0% | 87,5% | 73,0% | 75,0% | 95,5% |
| Sens/Spef für cut-off 10 | 67,0% | 68,8% | 68,7% | 68,8% | 56,5% | 68,8% | 97,5% |
| Sens/Spef für cut-off 20 | 44,3% | 68,8% | 43,5% | 68,8% | 40,0% | 62,5% | 100,0% |
| NT-ProBNP (pmol/ml), cut off 200 | 58,5% | 62,0% | 61,5% | 65,0% | 66,5% | 70,0% | 94,5% |
| Kombination: Copeptin > 7,5 pmol/l und/ oder NT-ProBNP >200pmol/ml | 81,2% | 93,5% | 83,5% | 96,0% | 79,5% | 84,5% | 93,8% |

### Probengewinnung, Biomarker-Analytik:

Blutabnahmen erfolgten mittels Standard-Serum-Monovetten. Nach einer Gerinnungszeit von 20-40 min erfolgte eine Zentrifugation für 15 min bei 2000 g die anschließende Serumtrennung erfolgte durch Dekantieren. Bis zur weiteren Verwendung wurden die Serumproben bei -20 Grad C gelagert.

NT-ProBNP wurde nach Omland et al (Omland T, Persson A, Ng L, et al. N-terminal pro-B-type natriuretic peptide and long-term mortality in acute coronary syndromes. Circulation. 2002; 106: 2913-2918) mit einem Lumineszenzimmunoassay bestimmt.

Copeptin wurde mittels des Copeptin-Lumineszenzimmunoassays der Firma Brahms AG bestimmt. Die Copeptin-Bestimmungsmethode ist ausführlich in Morgenthaler NG et al Clin. Chem. 2006 Jan, 52(1),112-9 beschrieben. Zusammenfassend: 50 Mikroliter Probe werden in ein mit Copeptin-Antikörper (AK1) beschichtetes Röhrchen pipettiert und mit je 200 Mikroliter einer Lösung aus Akridiniumester-markierten anti Copeptin-Antikörper (AK2) gemischt und 2 Stunden bei Raumtemperatur inkubiert. Nach Entfernung von nicht gebundenem (freiem) markierten Antikörper mittels 4-maligen Waschen mit Waschlösung (Lumitest Waschlösung, Brahms AG) erfolgte die Bestimmung gebundem Akridiniumester-markierten Antikörper in einem Luminometer der Firma Berthold.

### Prognose (Vorhersage des Ereigniseintritts):

Es zeigte sich überraschend, dass MI-Patienten mit Ereignissen innerhalb der folgenden 180 Tage post-Hospitalisierung noch höhere Copeptin Konzentrationen aufweisen, als solche ohne Ereignisse. Schon innerhalb von 6 Stunden nach Eintritt von MI-Symptomen zeigt Copeptin am exemplarischen Cut Off (Schwellenwert) von 30 pmol/l ein relatives Risiko von ca. 1:2 an (Patienten mit einem Copeptin > 30 haben ein zweimal höheres Risiko für spätere Ereignisse, als Patienten < 30 pmol/l.

Erwartungsgemäß fallen die Konzentrationen nach therapeutischen Interventionen (Tag 2ff, Tabelle 2b) ab. Hier zeigt sich überraschend, dass Patienten mit späterem Ereignis eine deutlich geringere Reduzierung des Biomarkers aufweisen. Dieses mündet in einer starken Erhöhung des Risikos auf Werte von bis zu 1:7,14 bei Patienten mit einem höheren Copeptin Wert als 30 pmol/l.

**Tabelle 2a**

| | 0-2 h | 0-2 h | 2-4 h | 2-4 h | 4-6 h | 4-6 h |
|---|---|---|---|---|---|---|
| | +E. | o.E. | +E. | o.E. | +E. | o.E. |
| Copeptin | | | | | | |
| Patienten > 30 pmol/l | 62,5% | 36,5% | 62,5% | 35,7% | 62,5% | 27,8% |
| Relatives Risiko für Herzinsuffizienz oder Tod bis 180 Tage nach Entlassung aus dem Krankenhaus | **1,7** | | **1.75** | | **2,25** | |

**Tabelle 2b**

| | Tag 2 | Tag 2 | Tag 3 | Tag 3 | Tag 4 | Tag 4 | Tag 5 | Tag 5 |
|---|---|---|---|---|---|---|---|---|
| | +E. | o.E. | +E. | o.E. | +E. | o.E. | +E. | o.E. |
| Copeptin | | | | | | | | |
| Patienten > 30 pmol/l | 50,0% | 7,0% | 43,8% | 7,0% | 39,2% | 6,1% | 31,3% | 6,1% |
| Relatives Risiko für Herzinsuffizienz oder Tod bis 180 Tage nach Entlassung aus dem Krankenhaus | **7,14** | | **6,26** | | **6,42** | | **5.13** | |

Legende in Tabellen und Figuren: surv.= Überleben, E. = Event (Ereignis): Tod, Entwicklung einer schweren Herzinsuffizienz, cut-off = Schwellenwert in pmol/L, Sens= Sensitivität, Spef= Spezifität, o. = ohne.

### Neurophysin:

Gemäß früheren Beschreibungen (Pullan (supra)) wurde ein Radioimmunoassay für Neurophysin erstellt: Neurohypophysäres Neurophysin wurde isoliert und quantifiziert. Damit wurden Kaninchen immunisiert und so hochtitrige anti-Neurophysin Antiseren gewonnen. Für den Immunoassay wurde das hochtitrigste Antiserum in einer Konzentration von 1:100.000 eingesetzt. Gereinigtes Neurophysin wurde mit der Chloramin T-Methode radiojodiert und als Tracer im Assay eingesetzt. Als Standards dienten Verdünnungen von gereinigtem Neurophysin in normalem Pferdeserum. Der Assay wurde wie folgt durchgeführt: 50 µl Probe bzw. Standard wurden mit 100 µl Tracer (12.000 dpm pro Bestimmung) und 100 µl verdünntem anti-Neurophysin Antiserum gemischt und 24 Stunden bei 4°C inkubiert. Als Puffer wurde verwendet: 100 mM Natrium Phosphat, pH 7.5, 0.1 % BSA. An Antikörper gebundener Tracer wurde von freiem Tracer getrennt, indem 60% Ethanol zugesetzt wurden und dann 15 Minuten bei 4°C und 5.000 g zentrifugiert wurde. Der Überstand wurde verworfen, und die im Pellet verbliebene Radioaktivität wurde bestimmt. Die Auswertung erfolgte mithilfe der Software Multicalc. Der Assay hatte eine analytische Nachweisgrenze von 22 pg/ml. Der Assay hatte einen Messbereich bis 400 pg/ml. Mit dem Assay wurden Plasmaproben verschiedener Patienten, wie weiter unten erläutert, vermessen. Proben mit Messwerten >400 pg/ml wurden in geeigneten Verdünnungen vermessen, so dass Messwerte innerhalb des Messbereichs erzielt wurden.

### Myokardinfarkt / Diagnose

Von 66 Patienten mit akutem Herzinfarkt wurden spätestens 6 Stunden nach Eintreten des Herzinfarkts Proben gewonnen und es wurde Neurophysin gemessen. Zum Vergleich wurde Neurophysin in 200 gesunden Kontrollen bestimmt. Die Receiver-Operator-Characteristics Analyse für die Diagnose des Herzinfarkts ergab eine AUC von 0,95. Bei einem Cut-off-Wert von 213 pg/ml ergab sich eine Sensitivität von 62,6% bei einer Spezifität von 98%. Bei einem Cut-off-Wert von 136,1 pg/ml ergab sich eine Sensitivität von 84% bei einer Spezifität von 95%.

### Myokardinfarkt / Prognose

Von 66 Patienten mit akutem Herzinfarkt wurden spätestens 6 Stunden nach Eintreten des Herzinfarkts bzw. am zweiten Tag nach Infarkt Proben gewonnen und es wurde Neurophysin gemessen. Die Patienten wurden über einen Zeitraum von 360 Tagen beobachtet. In diesem Zeitraum hatten 58 Patienten kein adverses Ereignis, 8 starben oder wurden wegen Herzinsuffizizienz rehospitalisiert.

### Prognose für Tag 1 (<6 Stunden nach Eintreten des Infarkts):

Durch Receiver-Operator-Characteristics Analyse wurde der beste Cut-off-Wert (definiert als das größte Produkt aus Sensitivität und Spezifität) zur Prognose der Mortalität bzw. Rehospitalisierung wegen Herzinsuffizienz ermittelt: 777 pg/ml. Bei diesem Cut-off-Wert betrug die Sensitivität der Prognose 62,5%, die Spezifität betrug 73%. Die Likelihood Ratio eines adversen Ereignisses betrug bei einem Cut-off-Wert von 777 pg/ml: 2,3.

### Prognose für Tag 2:

Durch Receiver-Operator-Characteristics Analyse wurde der beste Cut-off-Wert (definiert als das größte Produkt aus Sensitivität und Spezifität) zur Prognose der Mortalität bzw. Rehospitalisierung wegen Herzinsuffizienz ermittelt: 261 pg/ml. Bei diesem Cut-off-Wert betrug die Sensitivität der Prognose 68,8%, die Spezifität betrug 73%. Die Likelihood Ratio eines adversen Ereignisses betrug bei einem Cut-off-Wert von 261 pg/ml: 2,6.

### Figuren:

Figur 1:
   Copeptin Werte von Patienten nach Myokardinfarkt. Plasmaproben wurden von 131 Patienten, wie angegeben, zu verschiedenen Zeiten nach Myokardinfarkt gewonnen. Zusätzlich sind Copeptin Werte von Gesunden dargestellt ("controls"; 200 Werte). Copeptin Werte der Gruppen sind als Box-Whiskers-Plots dargestellt.
Figur 2:
   Copeptin Werte von Patienten nach Herzinfarkt. Plasmaproben wurden, wie angegeben, zu verschiedenen Zeiten nach Myokardinfarkt gewonnen. Die Patienten wurden danach gruppiert, ob später Tod bzw. ReHospitalisierung wegen Herzinsuffizienz eintrat ("event"; 16 Patienten) oder nicht ("surv"; 115 Patienten). Zusätzlich sind Copeptin Werte von Gesunden dargestellt ("controls"; 200 Werte). Copeptin Werte der Gruppen sind als Box-Whiskers-Plots dargestellt.
Figur 3:
   Darstellung der Aminosäuresequenz von Preprovasopressin (164 AS) und den Teilpeptiden und Fragmenten aus proAVP (AS: 29-164), Neurophysin II (AS: 32-124) und Copeptin (AS: 126-164).

## Patentansprüche

1. Verfahren zur Risikostratifizierung des akuten Koronarsyndroms, wobei eine Bestimmung von Copeptin oder Neurophysin II mittels einer in-vitro Diagnose erfolgt.

2. Verfahren zur Risikostratifizierung des akuten Koronarsyndroms nach Anspruch 1, zum Ermöglichen einer Identifizierung von Patienten mit erhöhtem Risiko oder/und einer ungünstigen Prognose eines akuten Koronarsyndroms, insbesondere Myokardinfarkt.

3. Verfahren zur Risikostratifizierung des akuten Koronarsyndroms nach Anspruch 1 oder 2, wobei der Patient ein symptomatischer oder asymptomatischer Patient, insbesondere ein Notfallpatient ist.

4. Verfahren zur Risikostratifizierung des akuten Koronarsyndroms nach einem der Ansprüche 1 bis 3
zum Ermöglichen einer Therapiesteuerung des akuten Koronarsyndroms, insbesondere Myokardinfarkts, insbesondere in der Intensivmedizin oder Notfallmedizin.

5. Verfahren zur Risikostratifizierung des akuten Koronarsyndroms nach einem der Ansprüche 1 bis 4 um Ermöglichen einer Durchführung von klinischen Entscheidungen, insbesondere weiterführende Behandlung und Therapie mittels Arzneimitteln, insbesondere in der Intensivmedizin oder Notfallmedizin.

6. Verfahren zur in-vitro Diagnostik zum Ermöglichen einer Frühdiagnose innerhalb von 6 Stunden nach Symptomauftritt oder Differentialdiagnose oder Prognose von Myokardinfarkt, wobei eine Bestimmung von Copeptin oder Neurophysin II von einem zu untersuchenden Patienten durchgeführt wird.

7. Verfahren zur Risikostratifizierung des akuten Koronarsyndroms nach Anspruch 1 oder Verfahren zur in-vitro Diagnostik zum Ermöglichen einer Frühdiagnose innerhalb von 6 Stunden nach Symptomauftritt oder Differentialdiagnose oder Prognose eines Myokardinfarkts nach Anspruch 6, wobei eine Bestimmung von Copeptin oder Neurophysin II in Kombination mit natriuretischen Proteinen, insbesondere ANP (bzw. ANF), proANP, NT-proANP, BNP, proBNP, NT-proBNP von einem zu untersuchenden Patienten durchgeführt wird.

8. Verfahren nach Anspruch 7, **dadurch gekennzeichnet, dass** die Diagnose zur Prophylaxe, zur Prognose, zur differentialdiagnostischen Früherkennung und Erkennung, zur Beurteilung des Schweregrades und zur therapiebegleitenden Verlaufsbeurteilung erfolgt.

9. Verfahren nach einem der vorherigen Ansprüche,
**dadurch gekennzeichnet,**
**dass** der cut-off (Schwellenwert) zur Diagnose 6-20 pmol/L, insbesondere 6-10 pmol/L oder der cut-off (Schwellenwert) zur Prognose 10-30 pmol/L, insbesondere 10-20 pmol/L der Marker Copeptin oder Neurophysin II signifikant (spezifisch) ist.

10. Verfahren nach einem der vorherigen Ansprüche,
**dadurch gekennzeichnet,**
**dass** zusätzlich eine Bestimmung mindestens eines weiteren Marker ausgewählt aus der Gruppe inflammatorischer Marker, cardiovaskulärer Marker, neurohormonaler Marker oder ischämischer Marker in einer in-vitro Diagnose durchgeführt wird.

11. Verfahren nach Anspruch 10, **dadurch gekennzeichnet, dass** der inflammatorische Marker aus mindestens einem Marker aus der Gruppe C-reaktivem Protein (CRP), Cytokinen, wie etwa TNF-alpha, Interleukinen, wie etwa IL-6, Procalcitonin (1-116, 3-116) und Adhäsionsmolekülen, wie VCAM oder ICAM ausgewählt ist.

12. Verfahren nach Anspruch 10, **dadurch gekennzeichnet, dass** der cardiovaskuläre Marker aus mindestens einem Marker aus der Gruppe Kreatinkinase, Myoglobin, Myeloperoxidase, natriuretisches Protein, insbesondere ANP (bzw. ANF), proANP, NT-proANP, BNP, proBNP, NT-proBNP oder jeweils eine Teilsequenz davon, kardiale Troponin, CRP sowie Kreisia-afregulierende (Pro)Hormone, wie pro-Gastrin-Releasing Peptid (proGRP), pro-Endothelin-1, pro-Leptin, pro-Neuropeptid-Y, pro-Somatostatin, pro- Neuropeptid-YY, pro-Opiomelanocortin oder pro-Adrenomedullin (proADM) ausgewählt ist.

13. Verfahren nach Anspruch 10, **dadurch gekennzeichnet, dass** der ischämische Marker aus mindestens einem Marker aus der Gruppe Troponin I und T, CK-MB ausgewählt ist.

14. Verfahren nach Anspruch 10, dadurch gekenntzeichnet, dass der neurohormonale Marker mindestens ein natriuretisches Protein ist, insbesondere ANP (bzw. ANF), proANP, NT-proANP, BNP, proBNP, NT-proBNP ist.

15. Verfahren nach einem der vorherigen Ansprüche,
**dadurch gekennzeichnet, dass** die Bestimmungen mittels einem Schnelltest, insbesondere in Einzel- oder Multiparameterbestimmungen durchgeführt werden.

16. Verfahren nach einem der vorherigen Ansprüche, **dadurch gekennzeichnet, dass** die Risilcostratifizierung zur Prognose, zur differentialdiagnostischen Früherkennung und Erkennung, zur Beurteilung des Schweregrades und zur therapiebegleitenden Verlaufsbeurteilung des akuten Koronarsyndroms erfolgt.

17. Verwendung von Copeptin oder Neurophysin II zur Risikostratifizierung des akuten Koronarsyndroms und/oder zur in-vitro Diagnostik zum Ermöglichen einer Frühdiagnose innerhalb von 6 Stunden nach Symptomauftritt oder Differentialdiagnose oder Prognose von Myokardinfarkt.

18. Verwendung eines Kits zur Risikostratifizierung des akuten Koronarsyndroms oder zur Frühdiagnose innerhalb von 6 Stunden nach Symptomauftritt oder Differentialdiagnose oder Prognose von Myokardinfarkt enthaltend Nachweisreagenzien zur Bestimmung von Copeptin oder Neurophysin II und ggfs. weitere Marker nach einem der Ansprüche 10 bis 14 und Hilfsmittel.

19. Kit zur Diagnose des akuten Koronarsyndroms, Myokardinfarkt enthaltend Nachweisreagenzien zur Bestimmung von Copeptin oder Neurophysin II in Kombination mit natriuretischen Proteinen, insbesondere ANP (bzw. ANF), proANP, NT-proANP, BNP, proBNP, NT-proBNP und ggfs. weitere Marker nach einem der Ansprüche 10 bis 14 und Hilfsmittel.

## Claims

1. A method for risk stratification for acute coronary syndrome, wherein copeptin or neurophysin II is determined by an *in vitro* diagnosis.

2. The method for risk stratification for acute coronary syndrome according to claim 1, for identification of patients with elevated risk and/or unfavourable prognosis of acute coronary syndrome, in particular myocardial infarction.

3. The method for risk stratification for acute coronary syndrome according to claim 1 or 2, wherein the patient is a symptomatic and/or asymptomatic patient, in particular an emergency care patient.

4. The method for risk stratification for acute coronary syndrome according to one of claims 1 to 3, for therapeutic control of acute coronary syndrome, in particular myocardial infarction, in particular in intensive or emergency medical care.

5. The method for risk stratification for acute coronary syndrome according to any one of claims 1 to 4, for making clinical decisions, in particular for further treatment and therapy with medicaments, in particular in intensive or emergency medical care.

6. A method for *in vitro* diagnostics for early diagnosis within 6 hours after the appearance of symptoms, or differential diagnosis, or prognosis of myocardial infarction, wherein copeptin or neurophysin II is determined in a patient to be studied.

7. The method for risk stratification for acute coronary syndrome according to claim 1 or the method for *in vitro* diagnostics for early diagnosis within 6 hours after the appearance of symptoms, or differential diagnosis, or prognosis of myocardial infarction according to claim 6, wherein copeptin or neurophysin II in combination with natriuretic proteins, in particular ANP (or ANF), proANP, NT-proANP, BNP, proBNP, or NT-proBNP, is determined in a patient to be studied.

8. The method according to claim 7, **characterised in that** the diagnosis is made for prophylaxis, prognosis, differential diagnostic early detection and identification, severity assessment, and prognostic assessment in conjunction with therapy.

9. The method according any one of the preceding claims, **characterised in that**, for diagnosis, the cut-off (threshold value) of 6-20 pmol/L, in particular 6-10 pmol/L, or, for prognosis, the cut-off (threshold value) of 10-30 pmol/L, in particular 10-20 pmol/L, of the marker copeptin or neurophysin II is significant (specific).

10. The method according to any one of the preceding claims, **characterised in that** a determination is also carried out using at least one additional marker selected from the group of inflammatory markers, cardiovascular markers, neurohormonal markers, or ischaemic markers in an *in vitro* diagnosis.

11. The method according to claim 10, **characterised in that** the inflammatory marker is selected from at least one marker from the group comprising C-reactive protein (CRP), cytokines such as TNF-alpha, interleukins such as IL-6, procalcitonin (1-116, 3-116), and adhesion molecules such as VCAM or ICAM.

12. The method according to claim 10, **characterised in that** the cardiovascular marker is selected from at least one marker from the group comprising creatin kinase, myoglobin, myeloperoxidase, natriuretic protein, in particular ANP (or ANF), proANP, NT-proANP, BNP, proBNP, or NT-proBNP, or in each case a partial sequence thereof, cardiac troponin, or CRP, as well as circulation-regulating (pro)hormones such as progastrin-releasing peptide (proGRP), proendothelin-1, proleptin, proneuropeptide-Y, prosomatostatin, proneuropeptide-YY, proopiomelanocortin, or proadrenomedullin (proADM).

13. The method according to claim 10, **characterised in that** the ischaemic marker is selected from at least one marker from the group comprising troponin I and T, and CK-MB.

14. The method according to claim 10, **characterised in that** the neurohormonal marker is at least one natriuretic protein, in particular ANP (or ANF), proANP, NT-proANP, BNP, proBNP, or NT-proBNP.

15. The method according to any one of the preceding claims, **characterised in that** the determinations are carried out using a rapid test, in particular in single- or multi-parameter determinations.

16. The method according to any one of the preceding claims, **characterised in that** the risk stratification is carried out for prognosis, differential diagnostic early detection and identification, severity assessment, and prognostic assessment in conjunction with therapy for acute coronary syndrome.

17. Use of copeptin or neurophysin II for risk stratification for acute coronary syndrome and/or for *in vitro* diagnostics for early diagnosis within 6 hours after the appearance of symptoms, or differential diagnosis, or prognosis of myocardial infarction.

18. Use of a kit for risk stratification for acute coronary syndrome, or for early diagnosis within 6 hours after the appearance of symptoms, or differential diagnosis, or prognosis of myocardial infarction, containing detection reagents for determining copeptin or neurophysin II, and optionally additional markers according to any one of claims 10 to 14, and auxiliary agents.

19. The kit for diagnosis of acute coronary syndrome or myocardial infarction, containing detection reagents for determining copeptin or neurophysin II in combination with natriuretic proteins, in particular ANP (or ANF), proANP, NT-proANP, BNP, proBNP, or NT-proBNP, and optionally additional markers according to any one of claims 10 to 14, and auxiliary agents.

## Revendications

1. Procédé de stratification des risques du syndrome coronarien aigu, dans lequel une détermination de la copeptine ou de la neurophysine II a lieu au moyen d'un diagnostic in vitro.

2. Procédé de stratification des risques du syndrome coronarien aigu selon la revendication 1, destiné à la possibilité d'identification de patients avec un risque élevé et/ou d'un pronostic défavorable en faveur d'un syndrome coronarien aigu, notamment d'un infarctus du myocarde.

3. Procédé de stratification des risques du syndrome coronarien aigu selon la revendication 1 ou 2, dans lequel le patient est un patient symptomatique ou asymptomatique, notamment un patient en état d'urgence.

4. Procédé de stratification des risques du syndrome coronarien aigu selon l'une des revendications 1 à 3,
destiné à la possibilité d'un contrôle de la thérapie du syndrome coronaire aigu, notamment d'un infarctus du myocarde, en particulier en soins intensifs ou en médecine d'urgence.

5. Procédé de stratification des risques du syndrome coronarien aigu selon l'une des revendications 1 à 4, destiné à permettre l'exécution de décisions cliniques, notamment d'une poursuite d'un traitement et d'une thérapie au moyens de médicaments, en particulier en soins intensifs ou en médecine d'urgence.

6. Procédé de diagnostic in-vitro pour permettre un diagnostic précoce en l'espace de 6 heures après l'apparition des symptômes ou un pronostic d'un infarctus du myocarde, où une détermination de la copeptine ou de la neurophysine II d'un patient à examiner est effectuée.

7. Procédé de stratification des risques du syndrome coronarien aigu selon la revendication 1 ou procédé de diagnostic in-vitro pour permettre un diagnostic précoce en l'espace de 6 heures après l'apparition des symptômes ou un diagnostic différentiel ou un pronostic d'un infarctus du myocarde selon la revendication 6, où une détermination de la copeptine ou de la neurophysine II est effectuée en combinaison avec des protéines natriurétiques, notamment l'ANP (respectivement l'ANF), la proANP, la NT-proANP, la BNP, la proBNP, la NT-proBNP d'un patient à examiner.

8. Procédé selon la revendication 7, **caractérisé en ce que** le diagnostic pour la prophylaxie, pour le pronostic, pour la reconnaissance précoce par diagnostic différentiel et la reconnaissance ont lieu pour l'évaluation du degré de gravité et pour l'évaluation de la thérapie d'accompagnement.

9. Procédé selon l'une des revendications précédentes,
**caractérisé en ce**
**que** le seuil (valeur de seuil) des marqueurs de la copeptine ou de la neurophysine est significatif (spécifique) de 6 à 20 pmol/l, notamment de 6 à 10 pmol/l pour le diagnostic, ou le seuil (valeur de seuil) est significatif (spécifique) de 10 à 30 pmol/l, notamment de 10 à 20 pmol/l pour le pronostic.

10. Procédé selon l'une des revendications précédentes,
**caractérisé en ce**
**qu'**en outre une détermination d'au moins un marqueur supplémentaire choisi dans le groupe constitué des marqueurs inflammatoires, des marqueurs cardiovasculaires, des marqueurs neuro-hormonaux ou des marqueurs ischémiques est effectuée dans un diagnostic in-vitro.

11. Procédé selon la revendication 10, **caractérisé en ce que** le marqueur inflammatoire est choisi parmi au moins un marqueur du groupe des protéines C-réactives (CRP), des cytokines, comme, par exemple, la TNF-alpha, les interleukines, comme, par exemple, l'IL-6, la procalcitonine (1-116, 3-116) et des molécules d'adhésion, comme, par exemple, la VCAM ou l'ICAM.

12. Procédé selon la revendication 10, **caractérisé en ce que** le marqueur cardiovasculaire est choisi parmi au moins un marqueur du groupe de la créatinkinase, de la myoglobine, de la .myéloperoxydase, d'une protéine natriurétique, notamment de l'ANP (respectivement l'ANF), la proANP, la NT-proANP, la BNP, la proBNP, la NT-proBNP ou respectivement une séquence partielle de celles-ci, de la troponine cardiaque, de la CRP, ainsi que des pro-hormones régulant la circulation, comme le peptide libérant la pro-gastrine (proGRP), la pro-endothéline-1, la pro-leptine, le pro-neuropeptide-Y, la pro-somatostatine, le pro- neuropeptide-YY, la pro-opiomélanocortine ou la pro-adrénomédulline (proADM).

13. Procédé selon la revendication 10, **caractérisé en ce que** le marqueur ischémique est choisi parmi un marqueur du groupe de la troponine I et T, et la CK-MB.

14. Procédé selon la revendication 10, **caractérisé en ce que** le marqueur neuro-hormonal est au moins une protéine natriurétique, notamment l'ANP (respectivement l'ANF), la proANP, la NT-proANP, la BNP, la proBNP, la NT-proBNP.

15. Procédé selon l'une des revendications précédentes, **caractérisé en ce que** les déterminations sont effectuées au moyen d'un test rapide, notamment par des déterminations d'un paramètre individuel ou de paramètres multiples.

16. Procédé selon l'une des revendications précédentes, **caractérisé en ce que** la stratification des risques est effectuée pour le pronostic, pour la reconnaissance précoce par diagnostic différentiel et la reconnaissance, pour l'évaluation d'un degré de gravité et pour l'évaluation de la thérapie d'accompagnement du syndrome coronaire aigu.

17. Utilisation de la copeptine ou de la neurophysine II pour la stratification des risques du syndrome coronaire aigu et/ou pour le diagnostic in-vitro pour permettre un diagnostic précoce en l'espace de 6 heures après l'apparition des symptômes ou un diagnostic différentiel, ou un pronostic d'infarctus du myocarde.

18. Utilisation d'un kit pour la stratification des risques du syndrome coronaire aigu ou pour le diagnostic précoce en l'espace de 6 heures après l'apparition des symptômes, ou pour un diagnostic différentiel ou un pronostic d'un infarctus du myocarde, contenant des réactifs indicateurs pour la détermination de la copeptine ou de la neurophysine II, et éventuellement, d'autres marqueurs selon l'une des revendications 10 à 14 et des produits auxiliaires.

19. Kit pour le diagnostic du syndrome coronaire aigu, de l'infarctus du myocarde contenant des réactifs indicateurs pour la détermination de la copeptine ou de la neurophysine II en combinaison avec des protéines natriurétiques, notamment l'ANP (respectivement l'ANF), la proANP, la NT-proANP, la BNP, la proBNP, la NT-proBNP et éventuellement d'autres marqueurs selon l'une des revendications 10 à 14 et des produits auxiliaires.
